(19) **Europäisches Patentamt European Patent Office Office européen des brevets**

(11) **EP 4 744 655 A1**

(12) **EUROPEAN PATENT APPLICATION**
published in accordance with Art. 153(4) EPC

(43) Date of publication:
**20.05.2026 Bulletin 2026/21**

(21) Application number: **24838705.2**

(22) Date of filing: **05.07.2024**

(51) International Patent Classification (IPC):
**A61K 31/19** *(2006.01)* **A61P 25/00** *(2006.01)*

(52) Cooperative Patent Classification (CPC):
**A61K 31/19; A61K 31/295; A61K 31/315;
A61P 25/00; A61P 25/02**

(86) International application number:
**PCT/CN2024/103814**

(87) International publication number:
**WO 2025/011452 (16.01.2025 Gazette 2025/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **10.07.2023 CN 202310840161**

(71) Applicant: **Changchun Sinobiomaterials Co., Ltd.
Changchun, Jilin 130000 (CN)**

(72) Inventors:
• **WANG, Jinyue
Changchun, Jilin 130000 (CN)**
• **ZHUANG, Xiuli
Changchun, Jilin 130000 (CN)**
• **ZHANG, Tianhui
Changchun, Jilin 130000 (CN)**

(74) Representative: **Colombo, Stefano Paolo et al
Marchi & Partners S.r.l.
Via Vittor Pisani, 13
20124 Milano (IT)**

(54) **USE OF DIVALENT METAL LACTATE IN PREPARATION OF PRODUCT FOR REPAIRING NERVE DAMAGE**

(57) The present invention belongs to the technical field of biomedicine, and particularly relates to the use of a divalent metal lactate in the preparation of a product for preventing, improving, repairing and treating nerve damage. Preferably, the divalent metal lactate comprises one of or a combination of more than one of calcium lactate, magnesium lactate, zinc lactate and ferrous lactate. Provided in the present invention is a new treatment method for repairing central and peripheral nerve damage. The new treatment method has wide application prospects in the treatment and repair of central nerve damage caused by factors such as trauma, or peripheral neuritis or peripheral neurosensory abnormalities caused by factors such as viruses, diabetes and chemotherapeutic drugs.

EP 4 744 655 A1

**Description**

**TECHNICAL FIELD**

The present invention belongs to the technical field of biomedicine, and particularly relates to the use of a divalent metal lactate in the preparation of a product for preventing, improving, repairing and treating nerve damage.

**BACKGROUND**

The central and peripheral nervous systems constitute the complete nervous system of the human body. The nervous system plays a leading role in maintaining the homeostasis of the internal environment, preserving the integrity and unity of the body, and coordinating and balancing with the external environment. As society becomes more modernized, traffic accidents and sports-related incidents inevitably increase. At the same time, potential violent incidents and natural disasters can also lead to damage to the nervous system.

Spinal cord injury is a serious injury to the central nervous system that can impair sensory and motor function, placing a heavy burden on patients' families and society. Spinal cord injury is a complex and delicate process, which is divided into primary injury and secondary injury based on the pathological process of neuronal damage. The severity of spinal cord injury is mainly determined during the secondary injury stage. This is because the microenvironment of the spinal cord undergoes significant changes during the secondary injury process, including increased levels of reactive oxygen species, impaired blood vessels hindering oxygen transport leading to hypoxia, and increased inflammatory response. In addition, the nerves themselves have poor recovery capabilities, resulting in the inability of the spinal cord to regenerate naturally after injury. In clinical practice, surgery or rehabilitation training has limited efficacy for patients with spinal cord injuries and cannot effectively promote nerve regeneration and functional recovery. Due to the complex pathophysiological process following spinal cord injury, the existing single therapies in clinical practice have not been entirely satisfactory in the treatment of spinal cord injury. Currently, autologous nerve transplantation is considered the gold standard for treating nerve defects, but the limited availability of donor nerves and the functional defects of nerves in the donor area greatly limit its clinical application. Therefore, there is a continuous and urgent clinical need for safe, effective, inexpensive, and easy-to-administer drugs for the treatment of spinal cord injuries.

In addition, the repair of peripheral nerve injuries, especially long-distance defects, and the reconstruction of nerve function are also global challenges in clinical treatment. Autologous or allogeneic nerve transplantation is a commonly used clinical repair method for treating peripheral nerve injuries. However, it has a series of problems, such as limited donor sources, differences in nerve structure between donors and recipients, loss of nerve function in the donor area, scarring, and immune rejection. Peripheral neuritis and peripheral nerve sensory abnormalities are currently the most common peripheral nerve injuries in clinical practice. Peripheral neuritis, also known as peripheral neuritis or polyneuritis, is a dysfunction of the distal nerves of the limbs caused by various etiologies. Peripheral neuritis can be caused by a variety of factors, including nutritional metabolism, drugs and poisoning, vasculitis, tumors, trauma or mechanical compression. It is mainly manifested as abnormal sensory, motor and vegetative nerve function within the range innervated by the damaged nerve. The symptoms of peripheral neuritis include hypaesthesia, pain, numbness or picotement, formication, burning sensation, and hyperesthesia, etc. Peripheral neuritis can be multiple or single, symmetrical or asymmetrical, and is one of the most common types of diseases of the nervous system. When the cause of peripheral neuritis is known, treatment targeting the cause can alleviate symptoms and restore damaged nerves. However, the treatment is less effective for peripheral neuritis with unknown causes or primary refractory peripheral neuritis. Data shows that approximately 40% of patients with peripheral neuritis will suffer from sequelae, resulting in a high rate of disability and seriously affecting their physical and mental health and quality of life. Currently, there are few clinical treatments for peripheral neuritis, and they mainly involve Western medicine. For example, in clinical practice, peripheral neuritis caused by various reasons can be treated with high doses of B vitamins, such as vitamins $B_1$, $B_6$, and $B_{12}$. For those experiencing significant pain, analgesics and sedatives should be used. For inflammatory demyelinating diseases, adrenocortical hormones such as methylprednisolone, dexamethasone, or hydrocortisone can be used. However, these drugs often have high toxicity and side effects if used for a long time, easily leading to drug dependence, and they are also difficult to achieve a complete cure.

In previous studies, the inventors discovered that during the degradation of poly-L-lactic acid (PLLA), the molecular structure of PLLA is gradually destroyed and slowly hydrolyzed into lactic acid. Lactic acid can induce human fibroblasts to increase collagen production, leading to an increase in collagen fibers in the dermis and producing a filling and repairing effect. Based on this, the inventors have further discovered that PLLA and lactic acid and their related lactate compounds have beneficial effects on repairing cartilage, connective tissue, tendons, fascia, nerves and other tissues (see CN202210028046.9). The present invention is a continuation of the previous work, and has discovered a new use of divalent metal lactates in the preparation of products for preventing, improving, repairing and treating nerve damage.

## SUMMARY OF THE INVENTION

In order to overcome the defects and shortcomings of the prior art, the present invention provides a new use for divalent metal lactates. During the research process, the inventors unexpectedly discovered that divalent metal lactates can effectively prevent, improve, repair, and treat damage to the central and peripheral nervous systems. Based on this discovery, we conducted in-depth research and completed the present invention.

To achieve the objectives of the present invention, the following technical solutions are adopted:

The present invention provides use of divalent metal lactates in the preparation of products for preventing, improving, repairing and treating nerve damage, wherein the tissue in which nerve damage occurs is selected from collagen-rich nerve tissues.

Optionally, in the above use, the nerve damage is central nerve damage or peripheral nerve damage, and the prevention, improvement, repair, and treatment of nerve damage is nerve regeneration, nerve number increase, and/or nerve repair.

Optionally, in the above use, the central nerve damage includes spinal cord injury or meningoencephalomyelitis, and the peripheral nerve damage includes peripheral nerve injury, peripheral neuritis or peripheral neuropathy.

Optionally, in the above use, the peripheral neuritis is selected from one or more of the following: facial neuritis, diabetic peripheral neuritis, viral peripheral neuritis, peripheral neuritis caused by chemotherapy drugs, or Guillain-Barré syndrome.

Optionally, in the above use, the symptoms of peripheral neuritis include hypaesthesia, pain, numbness or picotement, formication, burning sensation, and hyperesthesia.

Optionally, in the above use, the divalent metal lactate comprises one of or a combination of more than one of calcium lactate, magnesium lactate, zinc lactate and ferrous lactate.

Optionally, in the above use, the divalent metal lactate is selected from magnesium lactate or a combination of zinc lactate and magnesium lactate.

Preferably, in the composition, the ratio of magnesium lactate to zinc lactate is 1:10 to 10:1 by weight.

Preferably, in the composition, the ratio of magnesium lactate to zinc lactate is 1:10, 1:9, 1:8, 1:7, 1:6, 1:5, 1:4, 1:3, 1:2, 1:1, 2:1, 3:1, 4:1, 5:1, 6:1, 7:1, 8:1, 9:1 or 10:1 by weight.

Most preferably, in the composition, the ratio of magnesium lactate to zinc lactate is 4:1 by weight.

Optionally, in the above use, the product is selected from one or more of drugs, kits, health products, and medical devices.

The concentration of the divalent metal lactate in the product is 10-80 mmol/L, more preferably, the concentration of the divalent metal lactate in the product is 20-60 mmol/L.

Optionally, in the above use, the medical device comprises one of or a combination of more than one of medical adhesive tape, bandage, gauze, plaster, sponge, and medical sutures.

Optionally, in the above use, in the drug, the divalent metal lactate is the active ingredient, and the drug further comprises a pharmaceutically acceptable carrier or excipient.

Preferably, in the drug, divalent metal lactate is the only active ingredient.

Optionally, in the above use, the dosage forms of the drug include injections or topical preparations.

Preferably, the topical preparation comprises one of or a combination of more than one of ointments, creams, patches, sprays, solutions, and lotions.

Preferably, the product is administered by one or more of the following routes: intravenous injection, in situ injection, intramuscular injection, subcutaneous injection, oral administration, or topical application.

In addition, those skilled in the art will know that the manner of use of the product, as well as the dosage and volume of application, are related to the age, physical condition and disease of the subject, and can be determined by a clinician as appropriate.

Compared with the prior art, the present invention has the following advantages:

The present invention provides a new treatment method that is safe, effective, inexpensive, and easy to administer for repairing central and peripheral nerve damage. In particular, the present invention discovers the use of divalent metal lactates, comprising one of or a combination of more than one of calcium lactate, magnesium lactate, zinc lactate, and ferrous lactate, in the preparation of products for preventing, improving, repairing, and treating nerve damage. Therefore, the new treatment method has wide application prospects in the treatment and repair of central nerve damage caused by factors such as trauma, or peripheral neuritis or peripheral neurosensory abnormalities caused by factors such as viruses, diabetes and chemotherapeutic drugs.

## DETAILED DESCRIPTION OF THE INVENTION

The following provides a further explanation of the present invention by referring to specific examples. It should be understood that the specific examples described herein are for illustrative purposes only and are not intended to limit the scope of the invention.

If the specific techniques or conditions are not indicated in the examples, the techniques or conditions described in the literature in the art or the product instructions shall be followed. The reagents and instruments used without indicating the manufacturer are all conventional products that can be purchased through normal channels.

The experimental methods in the following examples are all conventional methods, unless otherwise specified. Unless otherwise specified, all test materials used in the following examples are commercially available products.

In the examples below, all experiments are repeated three times, with at least three samples in each group. The experimental data are statistically analyzed using SPSS21.0 software. For the statistical description of the measurement data, mean $\pm$ standard deviation (Mean $\pm$ SD) is used, and analysis of variance is used to compare the indicators between groups. $P < 0.05$ indicates a statistically significant difference. When $P < 0.05$, paired samples tests can be performed using the LSD-t test.

**Example 1: Effects of divalent metal lactate on sciatic nerve injury model animals**

1. Establishment of a sciatic nerve injury model and experimental grouping

Totally 40 SD rats (male and female in 1:1), 8 weeks old, weighing 200-220 g, were placed in a temperature-controlled room (22$\pm$2°C). The animals were anesthetized by intraperitoneal injection of 3.0% (w/v) sodium pentobarbital (0.2 ml/100 g). After complete anesthesia, the skin of the left buttock of the rat was prepared, and the prepared skin was disinfected with alcohol swabs and iodine swabs. An oblique incision was then made, and the left sciatic nerve was exposed by blunt dissection in the gluteal intermuscular space. The sciatic nerve was clamped three times with hemostatic forceps at a distance of 0.5 cm from the lower edge of the piriformis muscle (10 s each time, with a 10-s interval). The muscle next to the clamping points was marked with medical sutures, and the muscle and skin were immediately sutured, and disinfection was performed again. After the surgery, the animals were randomly divided into 4 groups of 10 each. The rats were housed in separate cages and then given intraperitoneal injections of drugs or normal saline according to the grouping once a day for 4 consecutive weeks. The experimental grouping and treatment methods are shown in Table 1. Postoperatively, the rats' general condition, skin ulceration around the incision, and the recovery of function of the limb on the operated side were closely observed.

**Table 1: Experimental grouping and treatment**

| Group | Group | Number of rats | Administration method | Dosage |
|---|---|---|---|---|
| Group A | Sciatic nerve injury group | 10 | No medication | The corresponding volume of normal saline |
| Group B | Sciatic nerve injury + low-dose magnesium lactate group | 10 | Intraperitoneal injection | 30 mmol/L |
| Group C | Sciatic nerve injury + medium-dose magnesium lactate group | 10 | Intraperitoneal injection | 150 mmol/L |
| Group D | Sciatic nerve injury + high-dose magnesium lactate group | 10 | Intraperitoneal injection | 250 mmol/L |

2. Footprint acquisition and sciatic nerve index scoring

A homemade rat gait recording box was constructed, with a channel length of 90 cm, a width of 15 cm, and a height of 20 cm. A 15 cm wide continuous recording paper was placed at the bottom. At 1, 2, 3 and 4 weeks postoperatively, rats were coated with a colored, non-toxic dye on the soles of their hind feet and walked on the continuous recording paper to leave bilateral footprints. Footprints of the experimental foot (E) and the normal foot (N) were selected, and the following variables were measured: ① print length (PL), which is the distance from the heel to the toe; ② toe spread (TS), which is the distance between the first and fifth toes; ③ inter toe distance (IT), which is the distance between the first and fourth toes. The above three variables are substituted into the following formula to calculate the sciatic nerve function index (SFI). When SFI=0, it is normal, and when SFI=-100, it is a complete injury. The calculation formula is as follows:

$$SFI=-38.3\times(EPL-NPL)/NPL+109.5\times(ETS-NTS)/NTS+13.3\times(EIT-NIT)/NIT-8.8$$

Footprints of rats were collected and SFI was calculated at various time points postoperatively (1 week, 2 weeks, 3 weeks and 4 weeks) to evaluate the motor function and recovery of rats in each group. The results are shown in Table 2. The results showed that at 2, 3 and 4 weeks postoperatively, the SFIs of the low-, medium- and high-dose magnesium lactate groups were significantly better than that of the normal saline-treated control group (Table 2, $P < 0.01$ for the medium-dose magnesium lactate group, $P < 0.05$ for the low- and high-dose magnesium lactate groups). This indicates that low, medium, and high doses of magnesium lactate have certain therapeutic effects and can promote the recovery of sciatic nerve function. Meanwhile, at 2, 3 and 4 weeks postoperatively, the SFI in the medium-dose magnesium lactate group was significantly better than that in the low-dose and medium-dose magnesium lactate groups ($P < 0.05$), indicating that medium-dose magnesium lactate has a better therapeutic effect on nerve injury and is more conducive to the recovery of nerve function.

**Table 2: Sciatic nerve index of different groups at different time points postoperatively (n=10)**

| Postoperative time (week(s)) | Group A | Group B | Group C | Group D |
|---|---|---|---|---|
| 1 | -76.35±6.36 | -74.03±7.31 | -72.95±6.81 | -73.99±7.06 |
| 2 | -69.55±5.93 | -64.52±7.25[a] | -55.38±5.03[aa] | -59.95±6.14[ab] |
| 3 | -53.78±5.34 | -49.47±5.56[a] | -39.17±5.38[aa] | -44.32±5.47[ab] |
| 4 | -44.82±4.77 | -40.71±90[a] | -30.99±3.65[aa] | -36.85±4.23[ab] |

Note: [a] indicates that compared with group A, $P < 0.05$; [aa] indicates that compared with group A, $P < 0.01$; [b] indicates that compared with groups B and D, $P < 0.05$.

3. Real-time quantitative PCR

3.1 Extraction of total RNA

The sciatic nerve was removed, washed in DEPC-treated water, 0.5 mL of Trizol was added, the mixture was ground thoroughly, centrifuged, and the supernatant was collected. 0.1 mL of chloroform was added, the mixture was centrifuged, and the supernatant was collected. 0.8 mL of isopropanol was added, the mixture was centrifuged, and the supernatant was discarded. 1 mL of 75% ethanol was added, the mixture was centrifuged, and the supernatant was discarded. 20 µL of DEPC-treated water was added to dissolve the RNA. The concentration and purity of the total RNA solution were determined using a nucleic acid protein detector.

3.2 Real-time quantitative PCR reaction

The expression of Caspase-3 and GAP-43 in sciatic nerve tissue of each group was detected by RT-qPCR.

Primer design

    Caspase-3 upstream primer 5'-GAGCTTGGAACGGTACGATA-3'
    Downstream primer 5'-CCGTACCAGAGCGAGATGAC-3'
    GAP-43 upstream primer 5'-GTGTGTGAGCCTGTCCTCTC-3'
    Downstream primer 5'-AAAACCGGGGTACAGTGCAA-3'

Caspase-3 plays a key role in apoptosis and is also a potential drug target. Studies have shown that Caspase-3 can severely inhibit DNA replication, transcription, and damage repair functions, ultimately leading to irreversible apoptosis. Therefore, reducing the activation of Caspase-3 can effectively inhibit apoptosis. The results of real-time quantitative PCR for Caspase-3 are shown in Table 3. The results showed that the expression of Caspase-3 mRNA in the normal saline control group did not change significantly at any time point. Compared with the control group for the same time point, the expression levels of Caspase-3 mRNA in the low-, medium- and high-dose magnesium lactate groups were significantly downregulated on day 1, day 3 and day 7 after modeling ($P < 0.05$). Meanwhile, within 7 days after modeling, the levels of Caspase-3 mRNA in the low-, medium-, and high-dose magnesium lactate groups gradually decreased over time ($P < 0.05$). Compared with the low-dose group, the expression levels of Caspase-3 mRNA in the medium- and high-dose magnesium lactate groups were significantly downregulated on day 1, day 3 and day 7 ($P < 0.05$). Compared with the high-dose group, the expression levels of Caspase-3 mRNA in the medium-dose magnesium lactate group were significantly lower at all time points. ($P < 0.05$). After the formation of sciatic nerve injury in rats, the expression of Caspase-3 in neurons is significantly enhanced, and the number of neuronal apoptosis also increases accordingly. Magnesium lactate injection can reduce Caspase-3 protein levels and decrease apoptosis caused by nerve damage.

**Table 3: Comparison of Caspase-3 mRNA expression at different time points after modeling and postoperatively**

| Group | Number of animals | Day 1 | Day 3 | Day 7 |
|---|---|---|---|---|
| Group A | 10 | 1.77±0.03 | 1.79±0.16 | 1.82±0.05 |
| Group B | 10 | 1.26±0.09 | 1.18±0.25[ac] | 1. 12±0.22[af] |
| Group C | 10 | 0.91±0.15[abd] | 0.79±0.21[abdc] | 0.62±0.15[abdf] |
| Group D | 10 | 1.41±0.06[ab] | 1.33±0.16[abc] | 1.21±0.22[abf] |

Note: Compared with group A, [a]$P < 0.05$; compared with group B, [b]$P < 0.05$; compared with group D, [d]$P < 0.05$; for comparison between day 1 and day 3 of the same group, [c]$P < 0.05$; for comparison between day 7 and day 3 of the same group, [f]$P < 0.05$.

GAP-43 is a membrane phosphoprotein widely found in neurons and is one of the markers of neuronal regeneration. It plays a key role in neuronal regeneration by regulating the actin cytoskeleton. The real-time quantitative PCR results of GAP-43 are shown in Table 4. The results showed that the expression of GAP-43 mRNA in the normal saline control group did not change obviously. Compared with the control group for the same time point, the expression levels of GAP-43 mRNA in the low-, medium- and high-dose magnesium lactate groups increased significantly on day 1, day 3 and day 7 after modeling ($P < 0.05$). Meanwhile, within 7 days after modeling, the levels of GAP-43 mRNA in the low-, medium-, and high-dose magnesium lactate groups gradually increased over time, reaching the highest level on day 7 ($P < 0.05$). Compared with the low-dose group, the expression levels of GAP-43 mRNA in the medium- and high-dose magnesium lactate groups were significantly upregulated on day 1, day 3 and day 7 ($P < 0.05$). Compared with the high-dose group, the expression levels of GAP-43 mRNA in the medium-dose magnesium lactate group increased significantly at all time points. ($P < 0.05$). Magnesium lactate can increase the expression of GAP-43, alleviate sciatic nerve injury in rats, and promote nerve regeneration.

**Table 4: Comparison of GAP-43 mRNA expression at different time points after modeling and postoperatively**

| Group | Number of animals | Day 1 | Day 3 | Day 7 |
|---|---|---|---|---|
| Group A | 10 | 1.12±0.24 | 1.33±0.16 | 1.25±0.05 |
| Group B | 10 | 1.27±0.06 | 1.65±0.14[c] | 2.38±0.36[af] |
| Group C | 10 | 1.91±09[abd] | 2.59±0.29[abdc] | 3.52±0.21 [abdf] |
| Group D | 10 | 1.76±05[ab] | 2.12±0.11[abc] | 2.89±0.35[abf] |

Note: Compared with group A, [a]$P < 0.05$; compared with group B, [b]$P < 0.05$; compared with group D, [d]$P < 0.05$; for comparison between day 1 and day 3 of the same group, [c]$P < 0.05$; for comparison between day 7 and day 3 of the same group, [f]$P < 0.05$.

**Example 2: Effects of divalent metal lactate on spinal cord injury model animals**

1. Establishment of a rat spinal cord injury model

Totally 40 SD rats (male and female in 1:1), 8 weeks old, weighing 200-220 g, were placed in a temperature-controlled room (22±2°C). The animals were anesthetized by intraperitoneal injection of 3.0% (w/v) sodium pentobarbital (0.2 mL/100 g). After complete anesthesia, the anesthetized animals were fixed in a prone position, and underwent skin preparation and routine disinfection. A longitudinal incision (2-3 cm) was made along the midline of the back with the twelfth thoracic vertebra (T12) as the base point, and the skin was cut from the outside to the inside until the subcutaneous fascia was reached. The dorsal lamina of the rat's T9-11 vertebrae was accurately removed to expose the dura mater of the spinal cord. A spinal cord injury (SCI) model was created by clamping the middle part of the spinal cord with an aneurysm clip for 40 s. During the experiment, the rats in the spinal cord injury group were helped to urinate twice a day, once in the morning and once in the evening, by squeezing their bladders. The perineum was cleaned after urination and kept dry. Any limbs that were wet with urine were washed and dried promptly, and the animal's body position was frequently changed. In addition, to prevent infection, gentamicin (2000 U/kg·d) was routinely injected intramuscularly 3 days postoperatively. Postoperatively, the animals were randomly divided into 4 groups of 10 rats each. The rats were housed in separate cages and then given intraperitoneal injections of drugs or normal saline once a day for 4 consecutive weeks, according to the grouping. The experimental grouping and treatment methods are shown in Table 5.

**Table 5: Experimental grouping and treatment**

| Group | Group | Number of rats | Administration method | Dosage |
|---|---|---|---|---|
| Group A | Spinal cord injury group | 10 | No medication | The corresponding volume of normal saline |
| Group B | Spinal cord injury group + low-dose magnesium lactate | 10 | Intraperitoneal injection | 30 mmol/L |
| Group C | Spinal cord injury group + medium-dose magnesium lactate | 10 | Intraperitoneal injection | 150 mmol/L |
| Group D | Spinal cord injury group + high-dose magnesium lactate | 10 | Intraperitoneal injection | 250 mmol/L |

2. Motor system scoring (BBB scoring)

The BBB scoring method, a neurological function assessment method proposed by American researchers Basso et al., was used to evaluate limb function in rats after spinal cord injury. The method is as follows: the animal was placed in an open basin, the basin wall was gently tapped to make the animal crawl, and the animal's hip, knee and ankle joints were observed for their walking, evasive movements and their coordination. The results are listed in Table 6.

As shown in Table 6, rats in all groups showed varying degrees of functional recovery after treatment with magnesium lactate, with the medium-dose magnesium lactate group showing the fastest recovery. One day postoperatively, there was no statistically significant difference in BBB scores of rates among the different groups ($P > 0.05$); however, the BBB scores of the different groups gradually increased over time with gradually widened differences . By 4 weeks postoperatively, the medium-dose magnesium lactate group had the highest score, followed by the high-dose magnesium lactate group, then the low-dose magnesium lactate group, and the normal saline-treated control group had the lowest score. Except for the low-dose and high-dose magnesium lactate groups, which had no statistically significant difference ($P = 0.213$), all other groups had statistically significant differences ($P < 0.05$).

**Table 6: Preoperative and postoperative BBB scores of different groups**

| Group | Group A | Group B | Group C | Group D |
|---|---|---|---|---|
| Preoperative | 21.00±0.00 | 21.00±0.00 | 21.00±0.00 | 21.00±0.00 |
| 1 day postoperatively | 1.82±0.76 | 2.16±0.42 | 1.82±0.99 | 1.99±0.90 |
| 1week postoperatively | 6.16±1.18 | 5.99±0.90 | 7.82±0.76 | 6.82±1.61 |
| 2 weeks postoperatively | 8.49±1.06[c] | 9.32±1.22[c] | 12.49±1.53[ab] | 11.82±1.18 |
| 3 weeks postoperatively | 10.16±1.34[dc] | 12.32±1.22[dc] | 15.16±1.48[ab] | 13.29±1.38[ab] |
| 4 weeks postoperatively | 11.16±0.99[bdc] | 13.99±0.90[ac] | 17.66±1.22[abd] | 15.99±1.11[ac] |

Note: [a] indicates that compared with group A, $P < 0.05$; [b] indicates that compared with group B, $P < 0.05$; [c] indicates that compared with group C, $P < 0.05$; [d] indicates that compared with group D, $P < 0.05$.

3. Real-time quantitative PCR

A 0.5 cm section of the injured spinal cord was excised from the lesion area, and the expression of brain-derived neurotrophic factor (BDNF), glial fibrillary acidic protein (GFAP), and neuron-specific enolase (NSE) genes was measured by RT-PCR.

3.1 Extraction of total RNA

1mL was added to a homogenization tube. 100 mg of tissue was taken and placed in the homogenization tube, RNA extraction solution was added, the mixture was ground thoroughly and centrifuged, and the supernatant was collected for later use. 250 μL of chloroform was added, the mixture was centrifuged, and the supernatant was collected.

0.8 mL of isopropanol was added, the mixture was centrifuged, and the supernatant was discarded. 1 mL of 75% ethanol was added, the mixture was centrifuged, and the supernatant was discarded. 15 μL of RNase-free water was added to dissolve the RNA. The concentration and purity of the total RNA solution were determined using a nucleic acid protein detector.

3.2 Reverse transcription reaction

3.3 Real-time quantitative PCR reaction

Primer design

BDNF upstream primer 5'-GTCAAGTGCCTTTGGAGCCT-3'
Downstream primer 5'-CATGGGATTGCACTTGGTCTC-3'
GFAP upstream primer 5'-AGTCGGCGAGTTACCAGGAG-3'
Downstream primer 5'-TTAATGACCTCGCCATCCCG-3'
NSE upstream primer 5'-TATCCTGGAGAACAGCGAAGC-3'd
Downstream primer 5'-GACAAAGTCCTGGTAGAGTGCCC-3'

BDNF is the most widely distributed and abundant neurotrophic factor in the mammalian brain. It plays an important role in the normal growth, development and plasticity of synapses and can inhibit apoptosis by activating downstream pathways. Table 7 shows that, 1 week postoperatively, the expression of BDNF in the normal saline-treated control group differed significantly compared with the low-, medium-, and high-dose magnesium lactate groups (P < 0.05). There was no statistically significant difference in BDNF expression between the low-dose magnesium lactate group and the high-dose magnesium lactate group (P= 0.628). At 4 weeks postoperatively, there was no statistically significant difference in BDNF expression between the normal saline-treated control group and the low-dose magnesium lactate group (*P*= 0.820), while the differences between the other groups were statistically significant (*P* < 0.05). At 4 weeks postoperatively, the differences in GFAP and NSE expression among the four groups were statistically significant (*P* < 0.05). BDNF, as an important neurotransmitter, plays a crucial role in neuronal growth, differentiation, and the regulation of synaptic plasticity by binding to specific receptors and activating downstream pathways. As shown in Table 7, divalent metal lactates can increase the expression of BDNF, which can then reduce neuronal apoptosis after spinal cord injury by activating downstream anti-apoptotic pathways and participate in the process of neural remodeling.

**Table 7: Relative expression of BDNF in rat spinal cord at 1 and 4 weeks postoperatively**

| Group | Group A | Group B | Group C | Group D |
|---|---|---|---|---|
| 1 week | 1.00[bd] | 3.32±0.48 | 4.91±0.02[abd] | 3.19±0.41 |
| 4 weeks | 0.56±0.11[d] | 0.67±0.09[d] | 9.30±1.03[abd] | 6.01+0.43 |

Note: [a] indicates that compared with group A, *P* < 0.05; [b] indicates that compared with group B, *P* < 0.05; [d] indicates that compared with group D, *P* < 0.05.

GFAP expression is an important reference indicator for the growth, proliferation and differentiation of spinal cord nerve cells, and is used as a therapeutic target for acute spinal cord injury. Table 8 shows that, 1 week postoperatively, the expression of GFAP in the normal saline-treated control group differed significantly compared with the low-, medium-, and high-dose magnesium lactate groups (*P* < 0.05). At 1 week postoperatively, the differences in GFAP expression among the four groups were statistically significant (*P* < 0.05). At 4 weeks postoperatively, the differences in GFAP expression among the four groups were statistically significant (*P* < 0.05). GFAP is a specific marker protein of astrocytes. Astrocytes are the most abundant cells in the central nervous system, and therefore have a high expression level postoperatively. Compared with other groups, the medium-dose divalent lactate group showed significantly reduced GFAP expression, indicating that it inhibits astrocyte proliferation and thus glial scar formation. This process can promote axonal regeneration and neural remodeling.

**Table 8: Relative expression of GFAP in rat spinal cord at 1 and 4 weeks postoperatively**

| Group | Group A | Group B | Group C | Group D |
|---|---|---|---|---|
| 1 week | 1.00[bd] | 1.65±0.06[d] | 0.35±0.05[abd] | 0.43±0.07 |

(continued)

| Group | Group A | Group B | Group C | Group D |
|---|---|---|---|---|
| 4 weeks | 4.16±0.61[bd] | 6.44±0.56[d] | 0.96±0.02[abd] | 1.49±0.09 |

Note: [a] indicates that compared with group A, $P < 0.05$; [b] indicates that compared with group B, $P < 0.05$; [d] indicates that compared with group D, $P < 0.05$.

NSE is an enzyme that plays a key role in the respiration process within nerve cells and is widely found in brain tissue and other peripheral nerve cells. NSE can participate in the body's glycolysis metabolism. When the body suffers damage such as hypoxia, ischemia, or poisoning, NSE will be released and can enter the blood circulation as the blood-brain barrier is broken. Table 9 shows that, 1 week postoperatively, the expression of NSE in the normal saline-treated control group differed significantly compared with the low-, medium-, and high-dose magnesium lactate groups ($P < 0.05$). There was no statistically significant difference in NSE expression among the low-, medium-, and high-dose magnesium lactate groups at 1 week postoperatively ($P > 0.05$). At 4 weeks postoperatively, the differences in NSE expression among the four groups were statistically significant ($P < 0.05$). Medium and high doses of divalent lactate can increase NSE expression levels, with the medium-dose group showing a more significant effect on increasing NSE. Since NSE participates in the catalytic reactions of cellular glucose metabolism, increased NSE expression can improve cellular energy metabolism and promote the recovery of damaged cells.

**Table 9: Relative expression of NSE in rat spinal cord at 1 and 4 weeks postoperatively**

| Group | Group A | Group B | Group C | Group D |
|---|---|---|---|---|
| 1 week | 1.00 | 1.40±0.07[a] | 1.66±0.12[a] | 1.51±0.08[a] |
| 4 weeks | 1.85±0.21[bd] | 2.53±0.30[d] | 3.68±0.12[abd] | 3.02±0.27 |

Note: [a] indicates that compared with group A, $P < 0.05$; [b] indicates that compared with group B, $P < 0.05$; [d] indicates that compared with group D, $P < 0.05$.

## Example 3: Effects of divalent metal lactate on autoimmune neuritis model animals

1. Establishment of an autoimmune neuritis model and experimental grouping

Totally 40 SD rats (male and female in 1:1), 8 weeks old, weighing 200-220 g, were placed in a temperature-controlled room (22+2°C). A mixture of 200 $\mu$g of P0$_{180-199}$, 1 mg of H37Ra, 100 $\mu$L of normal saline, and 100 $\mu$L of IFA was emulsified thoroughly, and as the injection dose for one rat, injected into the subcutaneous tissue of the soles of both hind limbs of a rat (100 $\mu$L per sole). After successful modeling, rats in the groups were administered the corresponding drugs or normal saline by gavage. The treatment was conducted once a day for four consecutive weeks.

**Table 10: Experimental grouping and treatment**

| Group | Group | Number of rats | Administration method | Dosage |
|---|---|---|---|---|
| Group A | Autoimmune neuritis group | 10 | No medication | The corresponding volume of normal saline |
| Group B | Autoimmune neuritis group + low-dose magnesium lactate | 10 | Gavage | 20 mmol/L |
| Group C | Autoimmune neuritis group + medium-dose magnesium lactate | 10 | Gavage | 90 mmol/L |
| Group D | Autoimmune neuritis group + high-dose magnesium lactate | 10 | Gavage | 250 mmol/L |

2. Behavioral score

Rats were observed and their behavior was scored daily during the administration period. 0 point: No clinical symptoms; 5 points: Paralysis of limbs or death.

On day 1 after being given normal saline by gavage, some rats in the autoimmune neuritis group began to show varying degrees of neurological dysfunction symptoms, including lethargy, unclean fur, and redness and swelling of both hind paws. On day 5, all rats showed varying degrees of autoimmune neuritis symptoms, successively exhibiting loss of muscle tone, tail dragging, and paralysis. On day 8, the autoimmune neuritis symptoms in rats reached their peak, with the highest neurological severity score of 4.0±0.5. In the low-dose magnesium lactate group, some rats showed varying degrees of neurological dysfunction symptoms on day 6 after medication; on day 11, all rats exhibited varying degrees of autoimmune neuritis symptoms; on day 15, the autoimmune neuritis symptoms of the rats reached their peak, with the highest neurological severity score of 3.0 ± 0.5; thereafter, the autoimmune neuritis symptoms of the rats gradually alleviated; on day 28, the neurological severity score was 0-1. In the medium-dose magnesium lactate group, some rats showed varying degrees of neurological dysfunction symptoms on day 10 after medication; on day 16, all rats exhibited varying degrees of autoimmune neuritis symptoms; on day 21, the autoimmune neuritis symptoms of the rats reached their peak, with the highest neurological severity score of 2.0±0.5. Thereafter, the symptoms of autoimmune neuritis in the rats gradually subsided; on day 25, the neurological severity score was 0. In the high-dose magnesium lactate group, some rats showed varying degrees of neurological dysfunction symptoms on day 8 after medication; on day 12, all rats exhibited varying degrees of autoimmune neuritis symptoms; on day 16, the autoimmune neuritis symptoms of the rats reached their peak, with the highest neurological severity score of 2.5±0.5; thereafter, the autoimmune neuritis symptoms of the rats gradually alleviated; on day 33, the neurological severity score was 0-1. The above results indicate that magnesium lactate has a certain therapeutic effect on neurological deficits caused by autoimmune neuritis, with the medium-dose of magnesium lactate showing the most significant effect.

3. Detection of inflammatory factor levels

Current theories suggest that inflammatory factors are one of the factors that aggravate the condition of autoimmune neuritis. The characteristic cytokine IL-17 secreted by Th17 cells participates in the development of autoimmune diseases by inducing inflammatory cascades in target organs. Blood was collected from the posterior orbital venous plexus of rats and centrifuged, and the supernatant was collected. The concentrations of IL-17 and IFN-$\gamma$ in serum were measured using a microplate reader, and the results are listed in Table 11. Compared with the autoimmune neuritis group treated with normal saline by gavage, the serum IL-17 and IFN-$\gamma$ levels in rats treated with magnesium lactate by gavage were significantly reduced ($P < 0.05$). Compared with the low- and high-dose magnesium lactate groups, the medium-dose magnesium lactate group showed significantly lower levels of IL-17 and IFN-$\gamma$ ($P < 0.05$). This indicates that magnesium lactate can reduce the secretion of inflammatory cytokines caused by autoimmune neuritis, and the medium-dose magnesium lactate has the most significant effect.

**Table 11: Comparison of serum IL-17 and IFN-$\gamma$ levels in rats from different groups**

|  | IL-6 | IFN-$\gamma$ |
| --- | --- | --- |
| Group A | 152.34±7.93 | 121.65±5.08 |
| Group B | 121.33±7.68[a] | 105.74±5.93[a] |
| Group C | 105.09±14.77[abd] | 93.11±7.62[abd] |
| Group D | 140.43±9.43[ab] | 117.65±11.60[ab] |

Note: [a] indicates that compared with group A, $P < 0.05$; [b] indicates that compared with group B, $P < 0.05$; [d] indicates that compared with group D, $P < 0.05$.

In the aforementioned application, the other divalent metal lactates include calcium lactate, zinc lactate, ferrous lactate, and various combinations, whose results are similar to the results of lactate magnesium mentioned above. Therefore, their details will not be elaborated here.

**Example 4: Effects of divalent metal lactate on autoimmune encephalomyelitis model animals**

1. Establishment of an autoimmune encephalomyelitis model and experimental grouping

Totally 40 SD rats (male and female in 1:1), 8 weeks old, weighing 200-220 g, were placed in a temperature-controlled room (22+2°C). The $MOG_{35-55}$ polypeptide was diluted with PBS to 6 mg/mL and mixed with an equal volume of CFA. The mixture was mixed by repeatedly pushing and pulling a dual-channel connector connected with a 5 mL syringe to prepare an $MOG_{35-55}$ water-in-oil antigen emulsion. Experimental rats were immunized with antigens. Antigen emulsion was injected subcutaneously at multiple points on the back of the rat, with 4 points per rat, for a total of 0.2 mL of antigen emulsion ($MOG_{35-55}$ polypeptide content, 600 $\mu$g per rat). At 0 hour and 48 hours after the completion of antigen emulsion immunization, PTX was administered intraperitoneally as a sensitizer to increase the morbidity of the model. After successful modeling, rats in the groups were administered the corresponding drugs or normal saline by gavage. The treatment was conducted once a day for four consecutive weeks.

**Table 12: Experimental grouping and treatment**

| Group | Group | Number of rats | Administration method | Dosage |
|---|---|---|---|---|
| Group A | Autoimmune encephalomyelitis group | 10 | No medication | The corresponding volume of normal saline |
| Group B | Autoimmune encephalomyelitis group + low-dose magnesium lactate | 10 | Gavage | 20 mmol/L |
| Group C | Autoimmune encephalomyelitis group + medium-dose magnesium lactate | 10 | Gavage | 90 mmol/L |
| Group D | Autoimmune encephalomyelitis group + high-dose magnesium lactate | 10 | Gavage | 250 mmol/L |

2. Neurological severity score

Rats were observed and scored for neurological severity daily during the administration period. 0 point: No clinical symptoms; 5 points: Near death, or death.

On day 5 after being given normal saline by gavage, some rats in the autoimmune encephalomyelitis group began to show varying degrees of neurological dysfunction symptoms. On day 9, all rats showed varying degrees of autoimmune encephalomyelitis symptoms, successively exhibiting loss of muscle tone, tail dragging, and paralysis. On day 12, the autoimmune encephalomyelitis symptoms in rats reached their peak, with the highest neurological severity score of $3.5\pm0.5$. In the low-dose magnesium lactate group, some rats showed varying degrees of neurological dysfunction symptoms on day 7 after medication; on day 12, all rats exhibited varying degrees of autoimmune encephalomyelitis symptoms; on day 16, the autoimmune encephalomyelitis symptoms of the rats reached their peak, with the highest neurological severity score of $2.5\pm0.5$; thereafter, the autoimmune encephalomyelitis symptoms of the rats gradually alleviated; on day 28, the neurological severity score was 0-1. In the medium-dose magnesium lactate group, some rats showed varying degrees of neurological dysfunction symptoms on day 12 after medication; on day 18, all rats exhibited varying degrees of autoimmune encephalomyelitis symptoms; on day 23, the autoimmune encephalomyelitis symptoms of the rats reached their peak, with the highest neurological severity score of $1.5\pm0.5$. Thereafter, the symptoms of autoimmune encephalomyelitis in the rats gradually subsided; on day 26, the neurological severity score was 0. In the high-dose magnesium lactate group, some rats showed varying degrees of neurological dysfunction symptoms on day 10 after medication; on day 15, all rats exhibited varying degrees of autoimmune encephalomyelitis symptoms; on day 19, the autoimmune encephalomyelitis symptoms of the rats reached their peak, with the highest neurological severity score of $2.5\pm0.5$; thereafter, the autoimmune encephalomyelitis symptoms of the rats gradually alleviated; on day 31, the neurological severity score was 0-1. Magnesium lactate has a certain therapeutic effect on neurological deficits caused by autoimmune encephalomyelitis, with the medium-dose of divalent lactate showing the most significant effect.

3. Detection of inflammatory factor levels

After euthanizing the rats, spinal cord tissue was taken, homogenized with tissue lysis buffer, centrifuged, and the supernatant was collected. The concentrations of TNF-$\alpha$, IL-6, and IL-$\beta$ were measured, and the results are listed in Table 13. Compared with the autoimmune encephalomyelitis group treated with normal saline by gavage, the levels of TNF-$\alpha$, IL-6 and IL-$\beta$ in the spinal cord of the rats that received magnesium lactate by gavage decreased significantly (P < 0.05). Compared with the low- and high-dose magnesium lactate groups, the medium-dose magnesium lactate group showed significantly lower levels of TNF-$\alpha$, IL-6, and IL-$\beta$ *(P < 0.05)*. This indicates that magnesium lactate can reduce the secretion of inflammatory cytokines caused by autoimmune encephalomyelitis, thereby reducing the damage to the myelin tissue, and the medium-dose magnesium lactate has a more significant effect.

**Table 13: Comparison of TNF-$\alpha$, IL-6, and IL-$\beta$ levels in the spinal cord of rats in different groups**

| | TNF-$\alpha$ | IL-6 | IL-$\beta$ |
|---|---|---|---|
| Group A | $362.08\pm43.41$ | $133.54\pm9.93$ | $101.85\pm7.00$ |
| Group B | $296.87\pm28.78$[a] | $102.93\pm9.68$[a] | $85.74\pm7.93$[a] |
| Group C | $257.17\pm43.06$[abd] | $85.19\pm16.77$[abd] | $73.21\pm9.09$[abd] |

(continued)

| | TNF-$\alpha$ | IL-6 | IL-$\beta$ |
|---|---|---|---|
| Group D | 309.93±39.68[ab] | 110.54±11.43[ab] | 87.85±9.75[ab] |

Note: [a] indicates that compared with group A, $P < 0.05$; [b] indicates that compared with group B, $P < 0.05$; [d] indicates that compared with group D, $P < 0.05$.

Obviously, those skilled in the art can make various modifications and variations to the present invention without departing from the spirit and scope of the present invention. Thus, if these modifications and variations of the present invention fall within the scope of the claims of the present invention and their equivalents, the present invention also intends to include these modifications and variations.

**Claims**

1. Use of a divalent metal lactate in the preparation of a product for preventing, improving, repairing and treating nerve damage, wherein the tissue in which nerve damage occurs is selected from collagen-rich nerve tissues.

2. The use according to claim 1, wherein the nerve damage is central nerve damage or peripheral nerve damage, and the prevention, improvement, repair, and treatment of nerve damage is nerve regeneration, nerve number increase, and/or nerve repair.

3. The use according to claim 2, wherein the central nerve damage includes spinal cord injury or meningoencephalomyelitis, and the peripheral nerve damage includes peripheral nerve injury, peripheral neuritis or peripheral neuropathy.

4. The use according to claim 3, wherein the peripheral neuritis is selected from one or more of the following: facial neuritis, diabetic peripheral neuritis, viral peripheral neuritis, peripheral neuritis caused by chemotherapy drugs, or Guillain-Barré syndrome, and the symptoms of the peripheral neuritis include hypaesthesia, pain, numbness or picotement, formication, burning sensation, and hyperesthesia.

5. The use according to any one of claims 1 to 4, wherein the divalent metal lactate comprises one of or a combination of more than one of calcium lactate, magnesium lactate, zinc lactate and ferrous lactate.

6. The use according to claim 5, wherein the divalent metal lactate is selected from magnesium lactate or a combination of zinc lactate and magnesium lactate, preferably, in the composition, the ratio of zinc lactate to magnesium lactate is 1:10-10:1 by weight.

7. The use according to claim 1, wherein the product is selected from one or more of drugs, kits, health products, and medical devices; preferably, the concentration of the divalent metal lactate in the product is 5-250 mmol/L; more preferably, the concentration of the divalent metal lactate in the product is 30-150 mmol/L.

8. The use according to claim 7, wherein the medical device comprises one of or a combination of more than one of medical adhesive tape, bandage, gauze, plaster, sponge, and medical sutures.

9. The use according to claim 7, wherein in the drug, the divalent metal lactate is an active ingredient, and the drug further comprises a pharmaceutically acceptable carrier or excipient.

10. The use according to claim 7, wherein the dosage form of the drug comprises an injection or a topical preparation; preferably, the topical preparation comprises one of or a combination of more than one of ointments, creams, patches, sprays, solutions, and lotions; preferably, the administration mode of the product comprises one or more of the following: intravenous injection, in situ injection, intramuscular injection, subcutaneous injection, oral administration, or topical application.

## INTERNATIONAL SEARCH REPORT

| International application No. |
| --- |
| **PCT/CN2024/103814** |

**A. CLASSIFICATION OF SUBJECT MATTER**

A61K31/19(2006.01)i; A61P25/00(2006.01)i

According to International Patent Classification (IPC) or to both national classification and IPC

**B. FIELDS SEARCHED**

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

WPABS; CNTXT; VCN; VEN; ENTXT; DWPI; CNKI; CJFD; 读秀; DUXIU; 万方; WANFANG; Elsevier; Pubmed; SpringerLink; ISI Web of Science; STNext: 乳酸盐, 神经损伤, 神经再生, 神经数量增加, 神经修复, 脊髓损伤, 神经炎, 格林巴利综合征, 格林-巴利综合征, 古兰-巴雷综合征, 急性感染性多发性神经根炎, 急性炎症性脱髓鞘性多神经根病, 乳酸钙, 乳酸镁, 乳酸锌, 乳酸亚铁, 乳酸钡, 乳酸锰, 乳酸铜, lactate, nerve injury, neurologic damage, nerve damage, nerve regeneration, increased nerve count, nerve repair, spinal cord injury, neuritis, Guillain, Barre, acute infectious polyradiculitis, calcium lactate, magnesium lactate, zinc lactate, ferrous lactate, barium lactate, manganese lactate, copper lactate, cupric lactate

**C. DOCUMENTS CONSIDERED TO BE RELEVANT**

| Category* | Citation of document, with indication, where appropriate, of the relevant passages | Relevant to claim No. |
| --- | --- | --- |
| X | WO 2014093918 A1 (WARSAW ORTHOPEDIC, INC. et al.) 19 June 2014 (2014-06-19) claims 1, 8, and 13 | 1-10 |
| X | JOURDAIN, P. et al. "L-Lactate Protects Neurons against Excitotoxicity: Implication of an ATP-Mediated Signaling Cascade" *Scientific Reports*, Vol. 6, 19 February 2016 (2016-02-19), pp. 1-13 p. 1, abstract | 1-10 |
| X | 明晔等 (MING, Ye et al.). "补钙不能单一补"钙"" (Non-official translation: Calcium Supplemention Cannot be Done by Simply Supplementing "Calcium")" 中国保健营养 *(China Health Care & Nutrition)*, No. 3, 31 December 2006 (2006-12-31), pp. 72 and 73 page 73, middle column, 3rd-to-last paragraph | 1-5, 7-10 |

☑ Further documents are listed in the continuation of Box C. ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
| --- | --- |
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **13 October 2024** | **21 October 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/103814** |

### C.     DOCUMENTS CONSIDERED TO BE RELEVANT

| Category* | Citation of document, with indication, where appropriate, of the relevant passages | Relevant to claim No. |
| --- | --- | --- |
| A | FALCONER, J. C. et al. "Time Dependence of N-Acetyl-Aspartate, Lactate, and Pyruvate Concentrations Following Spinal Cord Injury" *Journal of Neurochemistry,* Vol. 66, No. 2, 31 December 1996 (1996-12-31), pp. 717-722 p. 717, abstract | 1-10 |

Form PCT/ISA/210 (second sheet) (July 2022)

## INTERNATIONAL SEARCH REPORT
### Information on patent family members

International application No.

**PCT/CN2024/103814**

| Patent document cited in search report | | | Publication date (day/month/year) | Patent family member(s) | | | Publication date (day/month/year) |
|---|---|---|---|---|---|---|---|
| WO | 2014093918 | A1 | 19 June 2014 | US | 2015320795 | A1 | 12 November 2015 |
| | | | | EP | 2931292 | A1 | 21 October 2015 |
| | | | | EP | 2931292 | B1 | 13 June 2018 |
| | | | | AU | 2013358968 | A1 | 02 July 2015 |
| | | | | AU | 2013358968 | B2 | 21 December 2017 |

Form PCT/ISA/210 (patent family annex) (July 2022)

**REFERENCES CITED IN THE DESCRIPTION**

**Patent documents cited in the description**

- CN 202210028046